# EUROPEAN PATENT SPECIFICATION

(11) **EP 0 808 215 B1**
(45) Date of publication and mention of the grant of the patent: **09.09.1998**
(21) Application number: 96901347.3
(22) Date of filing: 31.01.1996
(51) Int. Cl.: B01J 23/66, B01J 23/68, C07D 301/10

(54) **ALKYLENE OXIDE CATALYST AND PROCESS**
ALKYLENOXIDKATALYSATOR UND VERFAHREN
CATALYSEUR DE PREPARATION D'OXYDE D'ALKYLENE ET PROCEDE

(30) Priority: 01.02.1995 US 383020
(43) Date of publication of application: 26.11.1997
(73) Proprietor: SHELL INTERNATIONALE RESEARCH MAATSCHAPPIJ B.V., 2596 HR Den Haag (NL)
(72) Inventor: BUFFUM, John, Edward, Houston, TX 77065 (US); KOWALESKI, Ruth, Mary, Houston, TX 77094 (US); LOCKEMEYER, John, Robert, Houston, TX 77479 (US); MATUSZ, Marek, Houston, TX 77084 (US)
(86) International application number: EP9600444
(87) International publication number: WO9623585

(56) References cited:
- EP-A- 0 243 996
- EP-A- 0 327 356
- EP-A- 0 625 370
- US-A- 4 428 863

## Description

The invention relates to silver-containing catalysts suitable for the preparation of alkylene oxide, in particular ethylene oxide, and to the use of the catalysts. The catalysts are prepared using a unique ceramic catalyst carrier.

Catalysts for the production of ethylene oxide from ethylene and molecular oxygen are generally supported silver catalysts. Such catalysts are typically promoted with alkali metals. The use of small amounts of the alkali metals potassium, rubidium and cesium were noted as useful promoters in supported silver catalysts in U.S. Patent No. 3,962,136, issued June 8, 1976, and U.S. Patent No. 4,010,115, issued March 1, 1977. The use of other co-promoters, such as rhenium, or rhenium along with sulfur, molybdenum, tungsten and chromium is disclosed in U.S. Patent No. 4,766,105, issued August 23, 1988, and U.S. Patent No. 4,808,738, issued February 28, 1989. U.S. Patent No. 4,908,343, issued March 13, 1990, discloses a supported silver catalyst containing a mixture of a cesium salt and one or more alkali metal and alkaline earth metal salts.

U.S. Patent No. 4,897,498, issued January 30, 1990, discloses the use of silver-based, alkali-metal promoted, supported catalysts in the epoxidation of olefins having no allylic hydrogens.

The use of porous ceramic catalyst carriers has been previously described in a number of patents such as, for example, U.S. Patent No. 5,100,859, issued March 31, 1992, U.S. Patent No. 5,055,442, issued October 8, 1991, U.S. Patent No. 5,037,794, issued August 6, 1991, and U.S. Patent No. 4,874,739, issued October 17, 1989. Such catalyst carriers have a wide variety of potential applications in the catalytic field and are especially useful where the ceramic base is an alumina such as alpha alumina. While alpha alumina is often the preferred ceramic catalyst base, it is understood that other ceramic materials such as silica, silicon carbide, silicon nitride, magnesia, titania, spinels and cordierite, as well as other forms of alumina may be used. In further discussions catalysts based on alpha alumina are used as examples but the teachings herein are understood to have a more general application.

A catalyst support needs to possess, in combination, at least a minimum surface area on which the catalytic component may be deposited, high water absorption and crush strength. Often an increase in one means a reduction in another property. Thus, high crush strength may mean low porosity. Usually the balance is achieved by trial and error making the catalyst carrier art even more unpredictable than other chemical process art.

Carriers need to have a uniform degree of porosity and this may be achieved in a number of ways including the incorporation of burnout materials that are eliminated when the ceramic is fired to form the finished product. Typical burnout materials include charcoal, petroleum coke, ground walnut shells and the like. Such materials usually leave leachable residues that can significantly impair the performance of catalyst supported on the carriers. Furthermore, the actual content of such leachable residue varies widely from batch to batch so that predictability is unsatisfactory.

There is therefore a need to design catalysts in which one can have confidence with respect to the final property balance. The catalysts of the present invention have an excellent balance of crush strength, abrasion resistance, porosity and catalytic performance that make them ideal for a wide range of catalytic applications.

European Patent Application 0 327 356, published August 9, 1989, discloses a process for preparing silver-containing catalysts for the production of ethylene oxide, the carriers of which are made of alumina materials, carbonaceous materials well matched both in particle sizes and in relative proportions, a fluxing agent, a fluoride and a binder. Optional carbonaceous materials are petroleum coke, carbon, graphite, polyethylene, rosin and the mixtures thereof. Only petroleum coke and graphite are actually exemplified. The effect according to this disclosure is to provide catalysts with a high selectivity.

This invention relates to a catalyst suitable for the epoxidation of olefins having no allylic hydrogen, in particular ethylene, with molecular oxygen in the vapor phase which catalyst comprises a catalytically effective amount of silver, a promoting amount of alkali metal, optionally a promoting amount of rhenium and optionally a promoting amount of a rhenium co-promoter, supported on a carrier prepared by a process which comprises mixing particulate ceramic components with from 0.5 to 50 parts by weight, based on 100 parts by weight of the ceramic components, of polypropylene in the form of a powder having an average particle size of less than 400 micrometer and an ash content of less than 0.1% by weight, and then firing at a temperature sufficient to burn off the synthetic organic polymer, sinter the particulate component and form a carrier.

The thus prepared carrier material has a low metallic leachables content. As used herein, "metallic leachables" refers to sodium, potassium, calcium and/or aluminum impurities resulting from the burnout material. As used herein, "leachables" refers to the sum of the metallic leachables plus contributions from other additives, some of which may impart desired characteristics to the carrier and/or the catalyst.

It has been found that catalysts having this unique ceramic carrier have improved physical properties relative to catalysts having conventional carriers. These catalysts have improved crush strength and abrasion resistance.

Descriptions of the carrier, the catalyst prepared with the carrier and the use of the catalyst are provided in detail below.

### The Carrier

The amount of leachables is measured by boiling a standard amount of the finished carrier in a standard volume of 10% nitric acid for 30 minutes. This extracts the metallic components in the form of the soluble nitrates which may then be separated and analyzed for the residual metallic values. Some silica may also be extracted by this method but this is disregarded for the purposes of this exercise because of the variable contribution made by bond and ceramic components.

The amount of metallic leachables remaining after firing the carrier to remove the synthetic organic polymer burnout material and resulting therefrom is desirably less than 2000 ppm, preferably less than 1500 ppm, and most preferably less than 1000 ppm, expressed in terms of the amount of the leachable metal in the finished carrier.

In a preferred process for preparing the carrier for the catalyst of the present invention, the polymer particles have an average particle size of from 5 to 400 and more preferably from 10 to 300 and most preferably from 15 to 200 micrometer.

The polymer particles can have any desired configuration, but since the objective is to produce a carrier material with a high but uniform porosity, this is most efficiently achieved if the particles have a generally blocky, (that is more nearly spherical), configuration. The porosity is also influenced greatly by the particle size of the ceramic material particles from which it is made. Often indeed such particles and their sizes are the dominant factors and the particle size of the burnout material has only a marginal effect on the porosity.

The polymer can be any one of those that are exclusively organic and contain no significant amounts of residual inorganic material as a result of the method by which it was made. The polymers may be formed using an emulsion polymerization process or by mass polymerization, including suspension polymerization, which is often preferred since the polymer can be obtained in the form of very fine particles that are directly usable in the carrier preparation process of the invention. While such fine particles are readily obtainable by such techniques, they can also be provided by conventional comminution techniques.

The polypropylene burnout materials can also be used in conjunction with minor amounts of conventional burnout materials where the need for very low leachables is not quite so great and minor amounts of the residuals can be tolerated.

Burnout materials leave behind other materials commonly referred to as "ash" when ignited and this is used as a measure of the amount of metallic leachables left in a ceramic material after burnout. The amount of ash and metallic leachable material is quite constant for any particular organic polymeric burnout material and this is a significant advantage.

There may not be an exact correspondence between ash content and the amount of leachables in the carrier in view of the potential contribution to the leachables content of other components of the composition from which the carrier is formed including surfactants, temporary binders, bond materials, etc., and the like. These latter components are usually and preferably chosen with specific properties in mind and the leachables contributed are accepted as an unavoidable consequence. If the metallic leachables contributed by the burnout material vary widely, as is the case with traditional burnout materials such as ground walnut shells, the total leachables can vary unpredictably and often exceed the preferred maximum. The use of a burnout material with a known, stable amount of metallic leachables allows the selection of the other composition components to establish a metallic leachables level that will not exceed the desired maximum level.

As indicated above, it is possible to define the preferred burnout material both in terms of the ash residue upon ignition or in terms of the metallic leachables that may be extracted from the finished carrier. In the case of the ash content, the amount should clearly be minimized so far as possible and a maximum level of 0.1% by weight is preferred. More preferred is a maximum level of 0.05%.

The material from which the carrier is made is not critical and any ceramic-based carrier is adaptable to the use of the organic polymeric burnout materials. The carrier can be, for example, an alumina-based carrier of the kind described in U.S. Patents No. 5,266,548; 5,100,859; 5,055,042. Alternatively, it can be based on silica, aluminosilicates, cordierite, zirconia, spinels, magnesia, or titania as well as combinations of such materials. It is preferably based predominantly, (that is at least 90% by weight of the ceramic components), on alumina and particularly alpha alumina though minor amounts of other ceramic oxides, (calcia, magnesia, strontia, titania and zirconia), or silicates may be present.

An example of a process for the production of the preferred alumina-based catalyst carriers of the invention comprises:
i) forming a mixture comprising:
   a) at least one particulate alumina component;
   b) from 0.5 to 50%, based on the weight of the total ceramic components, of a synthetic organic burnout material having an ash content of less than 0.1%; and
   c)water in sufficient quantity to extrude the above mixture;
ii) extruding the mixture into the desired shapes; and
iii) firing to sinter the alumina particulates to produce a porous alpha alumina-based carrier with a surface area of from 0.40 to 5.0, and preferably from 0.40 to 1.5 m²/gm and less than 2000, preferably less than 1500 ppm metallic leachables.

The catalyst carrier of the present invention may comprise a number of alumina and optionally other ceramic-forming components chosen to contribute to the desired catalytic and/or physical properties, including porosity, pore volume, crush strength and the like. Often a combination of two different alpha aluminas is preferred, wherein one component having larger particles is mixed with a second component having smaller particles, in weight ratios of from 10:90 to 90:10. The objective of this is to end up with a surface area in the finished product of from 0.4 to 5 m²/gm. As used herein, "surface area" is understood to mean the BET surface area measured using nitrogen or krypton as the adsorbed gas. The surface area in the finished carrier is somewhat less than for the free alumina particles. Thus, a convenient mixture may comprise for example, two types of alpha alumina particles, the first having a surface area of about 1 m²/gm and the second having a surface area of about 3 to 5 m²/gm. Other components such as titania may often confer particular advantage to such carrier materials.

The preferred porous alpha alumina-based carriers for the catalyst of the present invention have a metallic leachables content below 2000 ppm. By comparison with a carrier having the same porosity and packing density made using the same ceramic components and with conventional or traditional non-polymeric organic burnout materials, they display an attrition that is at least 10%, and preferably at least 20%, lower and a crush strength that is at least 10%, and preferably at least 20%, higher.

The final calcined carrier preferably has a porosity of at least 50% and more desirably from 60% to 75%, a crush strength of at least 5 pounds, and a settled packing density of at least 0.5 kg per litre, preferably at least 0.6 kg per litre. The surface area of the final calcined carrier is preferably from 0.4 to 5, and more preferably from 0.6 to 1.5 m²/g.

It is often found advantageous to add titania to the mixture to be extruded in an amount that represents from 0.05% to 5.0%, preferably from 0.05% to 2.0%, and more preferably, from 0.08% to 1% of the weight of the fired carrier. Certain forms of alumina and bond material may also contain titania as impurities or components. The contribution of such forms of titania are not included in the amounts specified above. The titania can be added as the dioxide, as a titanate or as a precursor of titania. In the following description, all of the above options are understood to be included under the term "titania". Other similar materials such as, for example, zirconia or magnesia, may also be utilized.

The carrier alumina components can also be mixed with a binding agent and water, formed into shapes and calcined.

The term "binding agent" as used herein refers to an agent that holds together the various components of the carrier after they have been shaped into the final form, say by extrusion or pelleting. These binding agents allow the shaped materials to be dried and calcined without crumbling. Such binding agents are usually "sticky" organic materials such as polyvinyl alcohols or cellulosic materials. Binding agents may also serve as extrusion aids. In certain cases peptizing acids may be used in lieu of binding agents.

It is usually preferred to add a ceramic bond material to the mixture to give added strength to the fired carrier. Conventional ceramic bond materials are typically present in an amount of from about 0.01% by weight to about 5% by weight based on the total weight of the ceramic components, expressed as the oxides. Conventional ceramic bond materials can be used before and after firing. These materials typically comprise components, (expressed as the oxides), such as silica, alumina, alkali metal oxides, alkaline earth metal oxides, alkaline earth metal silicates, iron oxide and titanium oxide.

After the components of the carrier are mixed together, say by mulling, the mixed material is extruded into shaped pellets, for example, cylinders, rings, trilobes, tetralobes and the like. "Extrusion aids" such as Vaseline Petroleum Jelly and other organic lubricating materials may be used to facilitate extrusion. The extruded material is dried to remove water that could convert to steam during calcination and destroy the extrudate shapes. After drying to a low water content, i.e., less than about 2%, the extruded material is calcined under conditions sufficient to remove burnout agents, extrusion aids, and binding agents and to fuse the alpha alumina particles into a porous, hard mass. Calcination is typically carried out in an oxidizing atmosphere, say oxygen gas or preferably air and at a maximum temperature greater than about 1300 °C and preferably ranging from about 1350 °C to about 1500 °C. Times at these maximum temperatures typically range from about 0.1 to 10 hours, preferably from about 0.5 to 5 hours.

The calcined carriers and catalysts made therefrom will typically have pore volumes (water) ranging from about 0.2 to about 0.6 cc/g, preferably from about 0.3 to about 0.5 cc/g and surface areas ranging from about 0.15 to about 3 m²/g, preferably from about 0.3 to about 2 m²/g.

The carrier formulation preferably has a low soda content which is less than about 0.06% by weight. In practice it is very difficult to obtain a sodium-free formulation and soda contents from about 0.02 to 0.06% by weight are usually found acceptable.

The carriers described above are particularly suited for preparing ethylene oxide catalysts which have improved physical properties with respect to crush strength and abrasion resistance.

### The Catalyst

The catalysts of the present invention comprise a catalytically effective amount of silver and a promoting amount of alkali metal(s) deposited on a carrier prepared by a process as described above. Other promoters in promoting amounts may be optionally present such as rare earths, magnesium, rhenium and rhenium co-promoters selected from sulfur, chromium, molybdenum, tungsten, phosphorus, boron and mixtures thereof.

In general, the catalysts of the present invention are prepared by impregnating porous refractory supports comprising alpha alumina with silver ions or compound(s), complex(es) and/or salt(s) dissolved in a suitable solvent sufficient to cause deposition on the support of from 1 to 40, preferably from 1 to 30 percent by weight, basis the weight of the total catalyst, of silver. The impregnated support is then separated from the solution and the deposited silver compound is reduced to metallic silver. Also deposited on the support either prior to, coincidentally with, or subsequent to the deposition of the silver will be suitable ions, or compound(s) and/or salt(s) of alkali metal dissolved in a suitable solvent. Also deposited on the carrier coincidentally with the deposition of the silver and/or alkali metal will be suitable optional promoter compound(s), complex(es) and/or salt(s) dissolved in an appropriate solvent.

The catalysts of the present invention are prepared by a technique in which the alkali metal promoter as well as any additional promoters in the form of soluble salts and/or compounds are deposited on the catalyst and/or support prior to, simultaneously with, or subsequent to the deposition of the silver and each other. The preferred method is to deposit silver and alkali metal simultaneously on the support, that is, in a single impregnation step, although it is believed that the individual or concurrent deposition of the alkali metal prior to and/or subsequent to the deposition of the silver would also produce suitable catalysts.

Promoting amounts of alkali metal or mixtures of alkali metal are deposited on a porous support using a suitable solution. Although alkali metals exist in a pure metallic state, they are not suitable for use in that form. They are used as ions or compounds of alkali metals dissolved in a suitable solvent for impregnation purposes. The carrier is impregnated with a solution of alkali metal promoter ions, salt(s) and/or compound(s) before, during or after impregnation of the silver ions or salt(s), complex(es), and/or compound(s) has taken place. An alkali metal promoter may even be deposited on the carrier after reduction to metallic silver has taken place. The promoting amount of alkali metal utilized will depend on several variables, such as, for example, the surface area and pore structure and surface chemical properties of the carrier used, the silver content of the catalyst and the particular ions used in conjunction with the alkali metal cation, optional co-promoters. The amount of alkali metal promoter deposited upon the support or present on the catalyst generally lies between 10 and 3000, preferably between 15 and 2000 and more preferably, between 20 and 1500 parts per million by weight of total catalyst. Most preferably, the amount ranges between 50 and 1000 parts per million by weight of the total catalyst. For purposes of convenience, the amount of alkali metal deposited on the support or present on the catalyst is expressed as the metal. Without intending to limit the scope of the invention, it is believed that the alkali metal compounds are oxidic compounds.

In a preferred embodiment, at least a major proportion (greater than 50% wt.) of the alkali metals are selected from the group consisting of potassium, rubidium, cesium, and mixtures thereof. A preferred alkali metal promoter is cesium. A particularly preferred alkali metal promoter is cesium plus at least one additional alkali metal. The additional alkali metal is preferably selected from sodium, lithium and mixtures thereof, with lithium being preferred.

When a lithium compound is used as the additional alkali metal, the amount utilized is typically in the range of from 40 to 150, and preferably in the range of from 40 to 100 micromoles per gram, basis the total weight of the catalyst.

The catalyst may also contain moderating amounts of chloride for purposes of enhancing the startup procedure for the catalysts. When chloride is added to the catalyst, the carrier can be impregnated with a solution of chloride moderator ions, salt(s) and/or compound(s) before, during or after impregnation of the silver ions or salt(s), complex(es), and/or compound(s) has taken place and before, during or after impregnation of the promoter ions or salt(s), complex(es), and/or compound(s) has taken place. The chloride moderator may even be deposited on the carrier after reduction to metallic silver has taken place. Suitable chloride-containing salts used to prepare the impregnating solutions include promoter chlorides such as lithium chloride, sodium chloride, potassium chloride, rubidium chloride and cesium chloride as well as ammonium chloride. Ammonium chloride is a preferred salt for use in preparing the chloride-containing impregnating solutions. Other compounds which decompose to the chloride ion upon processing of the catalyst are also suitable. The chloride-containing impregnating solutions will normally contain at least a small amount of water to enhance solubility of the chloride-containing salt or compound. Other promoters and co-promoters can be used in conjunction with the silver and alkali metal promoters.

Non-limiting examples of other promoters include rhenium, sulfate, molybdate, tungstate and chromate (see U.S. Patent no. 4,766,105, issued August 23, 1988), as well as phosphate and borate; sulfate anion, fluoride anion, oxyanions of Groups 3b to 6b (see U.S. Patent no. 5,102,848, issued April 7, 1992); (i) oxyanions of an element selected from Groups 3 through 7b and (ii) alkali(ne) metal salts with anions of halides, and oxyanions selected from Groups 3a to 7a and 3b through 7b (see U.S. Patent no. 4,908,343, issued March 13, 1990).

Generally, the carrier is contacted with a silver salt, a silver compound, or a silver complex which has been dissolved in an aqueous solution, so that the carrier is impregnated with said aqueous solution; thereafter the impregnated carrier is separated form the aqueous solution, e.g., by centrifugation or filtration and then dried. The thus obtained impregnated carrier is heated to reduce the silver to metallic silver. It is conveniently heated to a temperature in the range of from 50 °C to 600 °C, during a period sufficient to cause reduction of the silver salt, compound or complex to metallic silver and to form a layer of finely divided silver, which is bound to the surface of the carrier, both the exterior and pore surface. Air, or other oxidizing gas, reducing gas, an inert gas or mixtures thereof may be conducted over the carrier during this heating step.

There are several known methods to add the silver to the carrier or support. The carrier may be impregnated with an aqueous solution containing silver nitrate dissolved therein, and then dried, after which drying step the silver nitrate is reduced with hydrogen or hydrazine. The carrier may also be impregnated with an ammoniacal solution of silver oxalate or silver carbonate, and then dried, after which drying step the silver oxalate or silver carbonate is reduced to metallic silver by heating, e.g., to 600 °C. Specific solutions of silver salts with solubilizing and reducing agents may be employed as well, e.g., combinations of the vicinal alkanolamines, alkyldiamines and ammonia. One such example of a solution of silver salts comprises an impregnating solution comprising a silver salt of a carboxylic acid, an organic amine solubilizing/reducing agent, and an aqueous solvent.

Suitable silver salts include silver carbonate and the silver salts of mono- and polybasic carboxylic and hydroxycarboxylic acids of up to about 16 carbon atoms. Silver carbonate and silver oxalate are particularly useful silver salts, with silver oxalate being most preferred.

An organic amine solubilizing/reducing agent is present in the impregnating solution. Suitable organic amine silver-solubilizing/reducing agents include lower alkylenediamines of from 1 to 5 carbon atoms, mixtures of a lower alkanolamine of from 1 to 5 carbon atoms with a lower alkylenediamine of from 1 to 5 carbon atoms, as well as mixtures of ammonia with lower alkanolamines or lower alkylenediamines of from 1 to 5 carbons. Four groups of organic amine solubilizing/reducing agents are particularly useful. The four groups include vicinal alkylenediamines of from 2 to 4 carbon atoms, mixtures of (1) vicinal alkanolamines of from 2 to 4 carbon atoms and (2) vicinal alkylenediamines of from 2 to 4 carbon atoms, mixtures of vicinal alkylenediamines of from 2 to 4 carbon atoms and ammonia, and mixtures of vicinal alkanolamines of from 2 to 4 carbon atoms and ammonia. These solubilizing/reducing agents are generally added in the amount of from about 0.1 to about 10 moles per mole of silver present.

One method of preparing the silver containing catalyst can be found in U.S. Patent 3,702,259. Other methods for preparing the silver-containing catalysts which in addition contain higher alkali metal promoters can be found in U.S. Patent 4,010,115 and U.S. Patent 4,356,312; U.S. Patent 3,962,136 and U.S. Patent 4,012,425. Methods for preparing silver-containing catalysts containing higher alkali metal and rhenium promoters can be found in U.S. Patent No. 4,761,394, and methods for silver-containing catalysts containing higher alkali metal and rhenium promoters and rhenium co-promoters can be found in U.S. Patent No. 4,766,105. Methods for preparing silver-containing catalysts with a variety of different promoters are found in U.S. patents 4,908,343, and 5,057,481.

A particularly preferred process of impregnating the carrier consists of impregnating the carrier with an aqueous solution containing a silver salt of a carboxylic acid, an organic amine and a salt of cesium and a salt of an additional alkali dissolved therein. Silver oxalate is a preferred salt. It can be prepared by reacting silver oxide (slurry in water) with (a) a mixture of ethylenediamine and oxalic acid, or (b) oxalic acid and then ethylenediamine, which latter is preferred, so that an aqueous solution of silver oxalate-ethylenediamine complex is obtained, to which solution is added a certain amount of cesium compound and a certain amount of an additional alkali metal compound. Other diamines and other amines, such as ethanolamine, may be added as well. A cesium-containing silver oxalate solution may also be prepared by precipitating silver oxalate from a solution of cesium oxalate and silver nitrate and rinsing with water or alcohol the obtained silver oxalate in order to remove the adhering cesium salt until the desired cesium content is obtained. The cesium-containing silver oxalate is then solubilized with ammonia and/or an amine in water. Rubidium-, potassium-, sodium-, lithium- and mixtures of alkali metal-containing solutions may be prepared also in these ways. The impregnated carriers are then heated to a temperature between 50 °C and 600 °C, preferably between 75 °C and 400 °C to evaporate the liquid and produce a metallic silver.

### The Process

In commercial operation, ethylene and oxygen are converted to ethylene oxide in an ethylene oxide reactor which comprises a large fixed tube heat exchanger containing several thousand tubes filled with catalysts. A coolant is used on the shell side of the reactor to remove the heat of reaction. Coolant temperatures are frequently utilized as an indication of catalyst activity, with high coolant temperatures corresponding to lower catalyst activities.

In the reaction of ethylene oxide with oxygen to produce ethylene oxide, the ethylene is typically present in at least a double amount (on a molar basis) compared with oxygen, but the amount of ethylene employed is generally much higher. The conversion is therefore conveniently calculated according to the mole percentage of oxygen which has been consumed in the reaction to form ethylene oxide and any oxygenated by-products. The oxygen conversion is dependent on the reaction temperature, and the reaction temperature is a measure of the activity of the catalyst employed. The value T₄₀ indicates the temperature at 40 percent oxygen conversion in the reactor and the value T is expressed in °C, and the value T_{1.5} indicates the temperature at 1.5 percent ethylene oxide production. This temperature for any given catalyst is higher when the conversion of oxygen is higher. Moreover, this temperature is strongly dependent on the employed catalyst and the reaction conditions. The selectivity (to ethylene oxide) indicates the molar amount of ethylene oxide in the reaction product compared with the total molar amount of ethylene converted. In this specification, the selectivity is indicated as S₄₀, which means the selectivity at 40 percent oxygen conversion, or as S_{1.5}, which means the selectivity at 1.5 percent ethylene oxide production.

The conditions for carrying out such an oxidation reaction in the presence of the silver catalysts according to the present invention broadly comprise those already described in the prior art. This applies, for example, to suitable temperatures, pressures, residence times, diluent materials such as nitrogen, carbon dioxide, steam, argon, methane or other saturated hydrocarbons, to the presence of moderating agents to control the catalytic action, for example, 1-2-dichloroethane, vinyl chloride, ethyl chloride or chlorinated polyphenyl compounds, to the desirability of employing recycle operations or applying successive conversions in different reactors to increase the yields of ethylene oxide, and to any other special conditions which may be selected in processes for preparing ethylene oxide. Pressures in the range of from atmospheric to about 3500 kPa (500 psig) are generally employed. Higher pressures, however, are not excluded. Molecular oxygen employed as reactant can be obtained from conventional sources. The suitable oxygen charge may consist essentially or relatively pure oxygen, a concentrated oxygen stream comprising oxygen in major amount with lesser amounts of one or more diluents, such as nitrogen and argon, or another oxygen-containing stream, such as air. It is therefore evident that the use of the present silver catalysts in ethylene oxide reactions is in no way limited to the use of specific conditions among those which are known to be effective. For purposes of illustration only, the following table shows the range of conditions that are often used in current commercial ethylene oxide reactor units and which are also suitable for the instant process.

**TABLE I**

| | |
|---|---|
| *GHSV | 1500-10,000 |
| Inlet Pressure | 1000-3500 kPa |

| Inlet Feed | |
|---|---|
| Ethylene | 1-40% |
| O₂ | 3-12% |
| Ethane | 0-3% |
| Chlorohydrocarbon moderator | 0.3-50 ppmv total |
| Argon and/or methane and/or nitrogen diluent | Balance |
| Coolant temperature | 180-315 °C |
| Catalyst temperature | 180-325 °C |
| O₂ conversion level | 10-60% |
| EO Production (Work Rate) | 32-320 kg EO/m³ |
| | catalyst/hr. |

| | |
|---|---|
| * Volume of gas at standard temperature and pressurepassing over one volume of packed catalyst per hour. | |

In a preferred application of the silver catalysts according to the invention, ethylene oxide is produced when an oxygen-containing gas is contacted with ethylene in the presence of the present catalysts at a temperature in the range of from about 180 °C to about 330 °C, and preferably a temperature in the range of from about 200 °C to about 325 °C.

While the catalysts of the present invention are preferably used to convert ethylene and oxygen to ethylene oxide, olefins having no allylic hydrogens can be oxidized using the silver catalysts of the present invention to produce a high selectivity of epoxide derivatives thereof by contacting the olefin feed with an oxygen-containing gas in the presence of an organic halide and the silver catalyst described above under defined oxidation conditions.

Olefins contemplated for use in this oxidation process are those which satisfy the following structural formula: 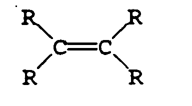 wherein each R is independently selected from the group consisting of:
(a) hydrogen,
(b) aryl and substituted aryl groups having in the range of 6 up to 20 carbon atoms,
(c) alkyl groups of the formula: 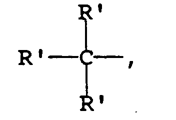 where each R' is independently: 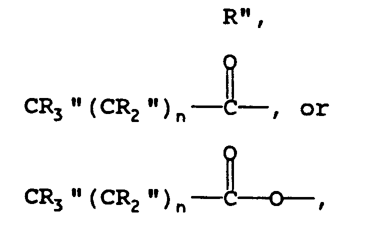 where R'' is H, C₁-C₁₀ alkyl or substituted alkyl, an aryl or substituted aryl group having 6 up to 20 carbon atoms, and n is a whole number from 0-12;
(d) CR₃''-(CR₂'')ₓ-O-, where x is a whole number from 1-12;
(e) 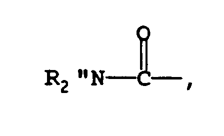
(f) R₂''N-;
(g) R''S-;
(h) CR₂''=CR''-(-CR''=CR''-)-_{y}, where y is an integer from 0-20; and
(i) 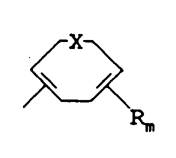 where X is 0, S or NR''; and m is an integer from 0-3 with the proviso that said olefin have no allylic hydrogens and that at least one R-group not be hydrogen.

Exemplary olefins which satisfy the above structural formula include butadiene, tertiary butylethylene, vinyl furan, methyl vinyl ketone, N-vinyl pyrrolidone, and the like. A presently preferred olefin for use in the practice of this process is butadiene because of its ready availability, relatively low cost, and the wide range of possible uses for the epoxide reaction product.

The epoxides produced by this process have the structural formula: 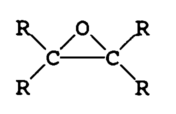 wherein each R is independently defined as set forth above. Where one or more of the R-groups contain carbon-carbon bond unsaturation, further oxidation can be carried out, thereby producing polyepoxide products.

The process is carried out by contacting the olefin to be oxidized with molecular oxygen and an organic halide under oxidation conditions, i.e. in the presence of sufficient quantities of an oxygen-containing gas to provide a molar ratio of olefin to oxygen in the range of 0.01 up to 20, and in the presence of 0.1 up to 1000 parts per million (by volume of total feed) of organic halide. Preferred quantities of organic halide for use in the practice of the present invention fall within the range of 1 up to 100 parts per million, by volume of total feed.

Suitable oxygen-containing gases include air, oxygen enriched air, substantially purified oxygen, oxygen diluted with inert gases such as N₂, Ar, CO₂, CH₄ and the like.

Suitable reaction temperatures fall within the range of 75 °C up to 325 °C. Preferred reaction temperatures fall within the range of 125 °C up to 295 °C; with temperatures in the range of 175 °C up to 290 °C being most preferred because selectivity to the desired epoxide falls off at temperatures significantly above about 290 °C and space-time yields are undesirably low at temperatures below about 175 °C.

The reaction pressure can vary within wide ranges, with typical limits of 9.807 - 9807 kPa (0.1 up to 100 atmospheres) being chosen primarily as a function of safety, handling, equipment, and other practical considerations. Preferably, reaction pressure is maintained in the range of 98.07 - 294.2 kPa (1 up to 30 atmospheres).

Reaction times suitable for this process can vary within wide ranges. Generally, olefin, oxygen, organic halide and catalyst are maintained in contact for a time sufficient to obtain olefin conversions per pass in the range of 0.1 up to 75 mole percent. Preferred target olefin conversion levels per pass fall within the range of 1 up to 50 mole percent, while reaction times sufficient to obtain olefin conversion per pass in the range of 5 up to 30 mole percent are presently most preferred for efficient utilization of the reactor capacity.

Those of skill in the art recognize that the actual contact times required to accomplish the desired conversion levels can vary within wide ranges, depending on such factors as vessel size, olefin to oxygen ratios, the silver loading level on the catalyst, the presence or absence of any catalyst modifiers (and their loading levels), the amount of organic halide present in the reaction zone, the reaction temperature and pressure, and the like.

The process can be carried out in either batch or continuous mode. Continuous reaction is presently preferred since high reactor throughput and high purity product is obtained in this manner. The batch mode is satisfactorily employed when high volume of reactant throughput is not required, for example, for liquid phase reactions.

For continuous mode of reaction carried out in the gas phase, typical gas hourly space velocities (GHSV) fall within the range of 100 up to 30,000 hr⁻¹. GHSV in the range of 200 up to 20,000 hr⁻¹ are preferred, with GHSV in the range of 300 up to 10,000 hr⁻¹ being most preferred because under such conditions the most desirable combination of feed olefin conversion and product selectivities are obtained.

When continuous mode of reaction is carried out in the liquid phase, typical liquid hourly space velocities (LHSV) employed will give contact times analogous to that obtained at the GHSV values given above. Most preferably, LHSV employed will fall in the range so as to produce the most desirable combination of feed olefin conversion levels and high product selectivity.

Recovery of the epoxide product produced can readily be carried out employing techniques well known by those of skill in the art. For example, where reaction is carried out in the continuous mode, unreacted starting material is initially separated from reaction products; and the desired product then isolated from the resulting product mixture by distillation, crystallization, extraction, or the like. Since the selectivity to the desired epoxide product is generally quite high, there are only small amounts of undesired reaction products from which to isolate the desired product.

Prior to use for oxidizing olefins having no allylic hydrogens, the silver catalysts (either before or after further treatment with promoter), are optionally calcined in an oxygen-containing atmosphere (air or oxygen-supplemented helium) at 350 °C for about 4 hours. Following calcination, the silver catalysts are typically subjected to an activation treatment at a temperature in the range of 300-350 °C in an atmosphere initially containing 2-5% hydrogen in an inert carrier such as helium or nitrogen. The hydrogen content of the activating atmosphere is gradually increased up to a final hydrogen concentration of 20-25% at a controlled rate so that the activation temperature does not exceed 350 °C. After the temperature is maintained for about 1 hour at a hydrogen concentration in the range of 20-25%, catalyst is ready for use.

More detailed descriptions of the silver catalysts and their use in oxidizing olefins having no allylic hydrogens are found in U.S. Patent Nos. 4,897,498, and 5,081,096.

The invention will be illustrated by the following illustrative embodiments.

### Illustrative Embodiments

### Carrier Preparation

The basic material of the following carriers was formulated as set forth in U.S. Patent No. 5,380,697. The burnout material in Carriers A, C and E was 30 parts of a particulate polypropylene having a maximum particle size of 150 micrometer, and an average particle size of about 90 micrometer. In Carriers B, D and F, the burnout material used was 42 parts of crushed walnut shells with an average particle size of 177 micrometer.

### Carrier A:

Carrier A was made using the formulations described below, and the procedure used was as follows:

The ceramic components, (270 parts of alpha alumina; 101 parts of gibbsite; 22 parts of boehmite), are mixed with the burnout material. to this mixture were added: 5 parts of an inorganic bond material; 15 parts of an organic binder; 7 parts of starch and 0.5 part of boric acid and the components were mixed for about 45 seconds. Following this mixing operation, the following components were added: water; 0.4 part of a surfactant, ("Triton" available under this trade name from Union Carbide Corporation); the titania-containing component, (0.6 part of an anatase with a surface area of about 16 m²/gm); and 8 parts of a fine alpha alumina seed component. The amount of water added was the amount necessary to make the mixture extrudable. Generally, this is about 120-125 parts by weight. The mixture is mixed for a further 4.5 minutes and then Vaseline is added to form an extrudable mixture (Vaseline is a trademark). The mixture is then mixed for a further 3.5 minutes before being extruded in the form of hollow cylinders and dried to less than 2% uncombined water. These were then fired in a tunnel kiln with a maximum temperature of about 1420-1425 °C for about 4 hours. The carrier is described in terms of its physical properties in Table 1.

### Carrier B:

Carrier B was prepared in a manner similar to Carrier A except that traditional burnout material, i.e. ground walnut shells, was added to the carrier formulation. The carrier is described in terms of its physical properties in Table 1.

### Carrier C:

Carrier C was prepared in a manner similar to Carrier A except that a water-soluble titania precursor (4.4 parts of a lactic acid chelate of titania containing about 0.6 part of titania) was used in the carrier instead of powdered titania, and the carrier was fired at a temperature of 1385-1390 °C. The carrier is described in terms of its physical properties in Table 1.

### Carrier D:

Carrier D was prepared in a manner similar to Carrier C except that traditional burnout material, i.e. ground walnut shells, was used. The carrier is described in terms of its physical properties in Table 1.

### Carrier E:

Carrier E was prepared in a manner similar to Carrier A except that the carrier contained no titania, and the carrier was fired at a temperature of 1470-1480 °C. The carrier is described in terms of its physical properties in Table 1.

### Carrier F:

Carrier F was prepared in a manner similar to Carrier E except that traditional burnout material, i.e. ground walnut shells, was used. The carrier is described in terms of its physical properties in Table 1.

**TABLE 1**

| CARRIER PROPERTIES | | | | | | |
|---|---|---|---|---|---|---|
| PROPERTY | Carrier A | Carrier B | Carrier C | Carrier D | Carrier E | Carrier F |
| Fired Temp. °C | 1420-5 | 1420-5 | 1385-9 | 1385-9 | 1470-8 | 1420-8 |
| Surface Area¹ | 1.01 | 0.94 | 1.09 | 1.23 | 1.13 | 1.02 |
| Pack. Den.² | 48.5 | 48.6 | 44.7 | 43.3 | 43.5 | 44.9 |
| Water Absorp.³ | 38.7 | 39.3 | 44.3 | 46.5 | 49.1 | 46.7 |
| Average C.S.⁴ | 15.6 | 12.7 | 11.3 | 7.7 | 18.9 | 17.1 |
| Attrition (%)⁵ | 6.8 | 10.4 | 19.3 | 25.6 | 16.2 | 18.8 |
| Leachable Na (ppm)⁶ | 395 | 472 | 641 | 592 | 348 | 682 |
| Leachable K (ppm)⁶ | 142 | 252 | 310 | 415 | 150 | 354 |
| Leachable Al (ppm)⁶ | 622 | 1014 | 1190 | 1204 | 642 | 1460 |
| Leachable Ca (ppm)⁶ | 224 | 378 | 354 | 497 | 220 | 518 |
| Burnout | Syn. | Trad. | Syn. | Trad. | Syn. | Trad. |

| | | | | | | |
|---|---|---|---|---|---|---|
| 1 "Surface Area" is the BET surface area measured using nitrogen or krypton as the adsorbate. | | | | | | |
| 2 "Packing Density" is the settled packing density as measured by ASTM D-4699-87, modified by the use of cylinder with an inside diameter of 3¾ inches and a length of 18 inches, or an equivalent. | | | | | | |
| 3 "Water Absorption" is a measure of the increase in weight of the carrier after being immersed in water and weighed. | | | | | | |
| 4 "Crush Strength" is measured on a Compton Tensile Tester, model 50-OP. | | | | | | |
| 5 "Attrition" is the attrition resistance and is the amount of carrier weight loss measured as a percentage using ASTM D-4058-92. | | | | | | |
| 6 "Leachables" were measured using the nitric acid solution technique. | | | | | | |

### Catalyst Preparation

The following illustrative embodiment describes preparative techniques for making the catalysts of the instant invention (Catalysts A, C and E) and the comparative catalysts (Comparative Catalysts B, D and F) and the technique for measuring the properties of these catalysts.

### Part A:Preparation of stock silver oxalate/ethylene-diamine solution for use in catalyst preparation:

1) Dissolve 415 grams (g) of reagent-grade sodium hydroxide in 2340 milliliters (ml) deionized water. Adjust the temperature to 50 °C.
2) Dissolve 1699 g of (high purity) silver nitrate in 2100 ml deionized water. Adjust the temperature to 50 °C.
3) Add sodium hydroxide solution slowly to silver nitrate solution with stirring while maintaining a temperature of 50 °C. Stir for 15 minutes after addition is complete, and then lower the temperature to 40 °C.
4) Insert clean filter wands and withdraw as much water as possible from the precipitate created in step (3) in order to remove sodium and nitrate ions. Measure the conductivity of the water removed and add back as much fresh deionized water as was removed by the filter wands. Stir for 15 minutes at 40 °C. Repeat this process until the conductivity of the water removed is less than 90 µmho/cm. Then add back 1500 ml deionized water.
5) Add 630 g of high-purity oxalic acid dihydrate in approximately 100 g increments. Keep the temperature at 40 °C and stir to mix thoroughly. Add the last portion of oxalic acid dihydrate slowly and monitor pH to ensure that pH does not drop below 7.8.
6) Remove as much water from the mixture as possible using clean filter wands in order to form a highly concentrated silver-containing slurry. Cool the silver oxalate slurry to 30 °C.
7) Add 699 g of 92 percent weight (%w) ethylene-diamine (8% deionized water). Do not allow the temperature to exceed 30 °C during addition.

The above procedure yields a solution containing approximately 27-33%w silver which provides the "stock solution" used in the preparation of Catalysts A, C and E and Comparative Catalysts B, D and F below.

### Part B: Preparation of impregnation solutions

### For Catalyst A:

To 153 grams of silver stock solution with a specific gravity of 1.526 was added 0.033 grams of NH₄F in 2 cc of water. 1.017 Grams of LiOH monohydrate was suspended in 5 grams of water, and the suspension was then added to the above silver solution. The solution was stirred until the LiOH was dissolved. CsOH (50% solution in water) in an amount of 0.1121 grams was added to 50 grams of the above silver solution and the resulting mixture was used for the carrier impregnation.

### For Comparative Catalyst B:

To 150 grams of silver stock solution with a specific gravity of 1.526 was added 0.032 grams of NH₄F in 2 cc of water. 1.007 Grams of LiOH monohydrate was suspended in 7 grams of water, and the suspension was then added to the above silver solution. The solution was stirred until the LiOH was dissolved. CsOH (50% solution in water) in an amount of 0.1132 grams was added to 50 grams of the above silver solution and the resulting mixture was used for the carrier impregnation.

### For Catalyst C:

To 181 grams of silver stock solution with a specific gravity of 1.565 was diluted with 1.3 grams of water. 0.035 Grams of NH₄F in 2 cc of water was added to the silver solution. 0.5497 Grams of LiOH monohydrate was dissolved in 20 grams of water and added to the above silver solution. CsOH (50% solution in water) in an amount of 0.1200 grams was added to 60 grams of the above silver solution and the resulting mixture was used for the carrier impregnation.

### For Comparative Catalyst D:

To 181 grams of silver stock solution with a specific gravity of 1.565 was diluted with 6.5 grams of water. 0.0034 Grams of NH₄F in 2 cc of water was added to the silver solution. 0.5316 Grams of LiOH monohydrate was dissolved in 20 grams of water and added to the above silver solution. CsOH (50% solution in water) in an amount of 0.1555 grams was added to 60 grams of the above silver solution and the resulting mixture was used for the carrier impregnation.

### For Catalyst E:

181 Grams of silver stock solution with a specific gravity of 1.565 was diluted with 19.6 grams of water and 12.6 grams of monoethanolamine. 0.033 Grams of NH₄F were dissolved in 2 cc of water and added to the silver solution. CsOH (50% solution in water) in an amount of 0.1117 grams was added to 60 grams of the above diluted silver solution and the resulting mixture was used for the carrier impregnation.

### For Comparative Catalyst F:

183 Grams of silver stock solution with a specific gravity of 1.555 was diluted with 12.5 grams of water and 13.2 grams of monoethanolamine. 0.034 Grams of NH₄F were dissolved in 2 cc of water and added to the silver solution. CsOH (50% solution in water) in an amount of 0.1137 grams was added to 60 grams of the above diluted silver solution and the resulting mixture was used for the carrier impregnation.

### Part C: Catalyst impregnation and curing

### Catalyst A:

Approximately 30 g of carrier A (described above in Table 1) is placed under 25 mm vacuum for 3 minutes at room temperature. Approximately 50 to 60 g of doped impregnating solution (as described in Part B above under "For Catalyst A") is then introduced to submerge the carrier, and the vacuum is maintained at 25 mm for an additional 3 minutes. At the end of this time, the vacuum is released, and excess impregnating solution is removed from the carrier by centrifugation for 2 minutes at 500 rpm. If the impregnating solution is prepared without monoethanolamine, then the impregnated carrier is then cured by being continuously shaken in a 849.5 l/hr.-(300 cu. ft/hr.) air stream flowing across a cross-sectional area of approximately 19.35 - 32.26 cm² (3-5 square inches) at 240-270 °C for 3-6 minutes. If significant monoethanolamine is present in the impregnating solution, then the impregnated carrier is cured by being continuously shaken in a 849.5 l/hr. (300 cu. ft./hr.) air stream at 250 °C to 270 °C for 4-8 minutes. The cured catalyst is then ready for testing.

The properties of Catalyst A are shown in Table 2 below.

### Comparative Catalyst B:

Comparative Catalyst B was prepared in the same manner as Catalyst A, except that Catalyst carrier B was used in place of Catalyst carrier A and the impregnating solution used was that described In Part B above under "For Comparative Catalyst B". The properties of Comparative Catalyst B are shown in Table 2 below.

### Catalyst C:

Catalyst C was prepared in the same manner as Catalyst A, except that Catalyst carrier C was used in place of Catalyst carrier A and the impregnating solution used was that described In Part B above under "For Catalyst C". The properties of Catalyst C are shown in Table 2 below.

### Comparative Catalyst D:

Comparative Catalyst D was prepared in the same manner as Catalyst A, except that Catalyst carrier D was used in place of Catalyst carrier A and the impregnating solution used was that described In Part B above under "For Comparative Catalyst D". The properties of Comparative Catalyst D are shown in Table 2 below.

### Catalyst E:

Catalyst E was prepared in the same manner as Catalyst A, except that Catalyst carrier E was used in place of Catalyst carrier A and the impregnating solution used was that described in Part B above under "For Catalyst E". The properties of Catalyst E are shown in Table 2 below.

### Comparative Catalyst F:

Comparative Catalyst F was prepared in the same manner as Catalyst A, except that Catalyst carrier F was used in place of Catalyst carrier A and the impregnating solution used was that described in Part B above under "For Comparative Catalyst F". The properties of Comparative Catalyst F are shown in Table 2 below.

**TABLE 2**

| CATALYST PROPERTIES | | | | | |
|---|---|---|---|---|---|
| | Ag (wt%) | Cs (ppm) | Li (µmol/g) | Attrition (%) | Crush Strength (gram) |
| Catalyst A | 15.1 | 540 | 80 | 8.8 | 7576 |
| Comp. Cat. B | 14.5 | 527 | 80 | 9.7 | 6486 |
| Catalyst C | 14.5 | 497 | 40 | 16.7 | 5261 |
| Comp. Cat. D | 14.5 | 666 | 40 | 17.4 | 4944 |
| Catalyst E | 14.5 | 500 | 0 | 8.9 | 8890 |
| Comp. Cat. F | 14.5 | 497 | 0 | 16.6 | 8255 |

As can be seen in Table 2, the Catalysts according to the invention (Catalysts A, C and E) have improved properties with respect to attrition resistance and crush strength when compared to the Comparative Catalysts not according to the invention (Comparative Catalysts B, D and F).

The actual silver content of the catalyst can be determined by any of a number of standard, published procedures. The actual level of cesium on the catalyst can be determined by employing a stock cesium hydroxide solution, which has been labeled with a radioactive isotope of cesium, in catalyst preparation. The cesium content of the catalyst can then be determined by measuring the radioactivity of the catalyst. Alternatively, the cesium content of the catalyst can be determined by leaching the catalyst with boiling deionized water. In this extraction process cesium, as well as other alkali metals, is measured by extraction from the catalyst by boiling 10 grams of whole catalyst in 20 milliliters of water for 5 minutes, repeating the above two more times, combining the above extractions and determining the amount of alkali metal present by comparison to standard solutions of reference alkali metals using atomic absorption spectroscopy (using Varian Techtron Model 1200 or equivalent).

### Part D: Standard Microreactor Catalyst Test Conditions/Procedure

### A. For Catalysts A and C and Comparative Catalysts B and D:

1 to 3 Grams of crushed catalyst (20-30 mesh, i.e. 0.841-0.595 mm) are loaded into a 6 mm diameter stainless steel U-shaped tube. The U tube is immersed in a molten metal bath (heat medium) and the ends are connected to a gas flow system. The weight of the catalyst used and the inlet gas flow rate are adjusted to achieve a gas hourly space velocity of 6800. The outlet gas pressure is 1550 kPa.

The gas mixture passed thorough the catalyst bed (in once-through operation) during the entire test run (including startup) consists of 25% ethylene, 7% oxygen, 5% carbon dioxide, 1.25 to 5 ppmv ethyl chloride with the balance being nitrogen/argon.

The startup procedure involved ramping the temperature from 180 °C to 230 °C in the following fashion: 1 hour at 180 °C, 1 hour at 190 °C, 1 hour at 200 °C, 1 hour at 210 °C, 1 hour at 220 °C, 2 hours at 220 °C, 2 hours at 225 °C, 2 hours at 230 °C, and then the temperature was adjusted to provide 1.5% ethylene oxide at the reactor outlet. Catalyst selectivity (S_{1.5}) and catalyst activity (T_{1.5}) were measured at those conditions.

To allow meaningful comparison of the performance of catalysts tested at different times, the Catalysts A and C, and Comparative Catalysts B and D were tested simultaneously with a standard reference catalyst which was S_{1.5} = 81.7% and T_{1.5} = 235 °C.

Catalysts A and C and Comparative Catalyst B and D prepared above were tested using the above procedure and the results are given in Table 3 below.

**TABLE 3**

| CATALYST PERFORMANCE | | |
|---|---|---|
| | S_{1.5}, % | T_{1.5}, °C |
| Catalyst A | 83.3 | 227 |
| Comparative Catalyst B | 83.1 | 228 |
| Catalyst C | 83.2 | 228 |
| Comparative Catalyst D | 83.3 | 232 |

### B. For Catalyst E and Comparative Catalyst F:

3 to 5 Grams of crushed catalyst (14-20 mesh, i.e. 1.410-0.841 mm) are loaded into a 6 mm diameter stainless steel U-shaped tube. The U tube is immersed in a molten metal bath (heat medium) and the ends are connected to a gas flow system. The weight of the catalyst used and the inlet gas flow rate are adjusted to achieve a gas hourly space velocity of 3300. The outlet gas pressure is 1550 kPa.

The gas mixture passed thorough the catalyst bed (in once-through operation) during the entire test run (including startup) consists of 30% ethylene, 8.5% oxygen, 5% carbon dioxide, 1.5 to 5 ppmv ethyl chloride with the balance being nitrogen/argon.

The catalysts were started in a manner similar to Catalysts A and C, and Comparative Catalysts B and D. Due to slight differences in feed gas composition, gas flow rates, and the calibration of analytical instruments used to determine the feed and product gas compositions, the measured selectivity and activity of a given catalyst may vary slightly from one test run to the next.

To allow meaningful comparison of the performance of catalysts tested at different times, all catalysts described in this illustrative embodiment were tested simultaneously with a standard reference catalyst which was S₄₀ = 81.0% and T₄₀ = 230 °C.

Catalyst E and Comparative Catalyst F prepared above were testing using the above procedure and the results are given in Table 4 below.

**TABLE 4**

| CATALYST PERFORMANCE | | |
|---|---|---|
| | S₄₀, % | T₄₀, °C |
| Catalyst E | 81.4 | 227 |
| Comparative Catalyst F | 81.3 | 228 |

## Claims

1. A catalyst suitable for the epoxidation of olefins having no allylic hydrogen, in particular ethylene, with molecular oxygen, which catalyst comprises a catalytically effective amount of silver and a promoting amount of alkali metal optionally a promoting amount of rhenium and optionally a promoting amount of rhenium copromoter deposited on a carrier which is prepared by a process which comprises mixing particulate ceramic components with from 0.5 to 50 parts by weight, based on 100 parts by weight of the ceramic components, of polypropylene in the form of a powder having an average particle size of less than 400 micrometer and an ash content of less than 0.1% by weight, and then firing at a temperature sufficient to burn off the synthetic organic polymer, sinter the particulate component and form a carrier.

2. The catalyst of claim 1 wherein, in the carrier, the polypropylene is present in an amount that is from 1 to 40% of the weight of the ceramic components.

3. The catalyst of claim 1 wherein, in the carrier, the ceramic components comprise at least 90% by weight of alpha alumina.

4. The catalyst of claim 1 wherein, in the carrier, the polypropylene has an ash content of less than 0.05% by weight.

5. The catalyst of claim 1 wherein, in the carrier, a ceramic bond material is added to the extrudable mixture in an amount that is from 0.01 to 5% of the weight of the ceramic components in the mixture.

6. The catalyst of claim 1 wherein the carrier comprises from 0.01 to 5% by weight, basis the total weight of the fired carrier, of a compound selected from the group consisting of alkaline earth metal oxide, silicon dioxide, zirconium dioxide and mixtures thereof.

7. The catalyst of claim 1 wherein the carrier further comprises from 0.05% by weight to 5% by weight, based on the weight of the fired carrier, of titania.

8. The catalyst of claim 1 wherein the silver ranges from 1 percent by weight to 40 percent by weight of the total catalyst and the alkali metal ranges from 10 parts per million to 3000 parts per million, expressed as the metal, by weight of the total catalyst.

9. The catalyst of claim 8 wherein said alkali metal promoter is selected from the group consisting of potassium, rubidium, cesium, lithium and mixtures thereof.

10. The catalyst of claim 9 wherein said promoter is cesium.

11. The catalyst of claim 1 wherein said alkali metal promoter comprises cesium plus at least one additional alkali metal.

12. The catalyst of claim 1 wherein the rhenium co-promoter is selected from the group consisting of sulfur, molybdenum, tungsten, chromium, phosphorus, boron and mixtures thereof.

13. A process for the production of ethylene oxide wherein ethylene is contacted in the vapor phase with an oxygen-containing gas at ethylene oxide forming conditions at a temperature in the range of from 180 °C to 330 °C in the presence of a catalyst according to any one of claims 1 to 12.

14. A process for the epoxidation of olefins having no allylic hydrogens wherein an olefin having no allylic hydrogen is contacted in the vapor phase with an oxygen-containing gas at epoxide forming conditions at a temperature in the range of from 75 °C to 325 °C in the presence of an organic halide and a catalyst according to any one of claims 1 to 12.

## Patentansprüche

1. Katalysator, der für die Epoxidation von Olefinen, die frei von Allylwasserstoff sind, insbesondere von Ethylen, mit molekularem Sauerstoff in der Dampfphase geeignet ist, welcher Katalysator eine katalytisch wirksame Menge an Silber, eine Promotormenge an Alkalimetall, gegebenenfalls eine Promotormenge an Rhenium und gegebenenfalls eine Promotormenge an einem Rheniumcopromotor, aufgebracht auf einen Träger, umfaßt, hergestellt nach einem Verfahren, das ein Mischen von teilchenförmigen keramischen Komponenten mit 0,5 bis 50 Gew.-Teilen, bezogen auf 100 Gew.-Teile der keramischen Komponenten, Polypropylen in Form eines Pulvers mit einer mittleren Teilchengröße von kleiner als 400 µm und mit einem Aschegehalt von weniger als 0,1 Gew.-%, anschließend ein Brennen bei einer Temperatur, die zum Abbrennen des synthetischen organischen Polymers ausreicht, ein Sintern der teilchenförmigen Komponenten und das Ausbilden eines Trägers umfaßt.

2. Katalysator nach Anspruch 1, worin in dem Träger das Polypropylen in einer Menge vorliegt, die 1 bis 40 % des Gewichtes der keramischen Komponenten ausmacht.

3. Katalysator nach Anspruch 1, worin in dem Träger die keramischen Komponenten wenigstens 90 Gew.-% α-Aluminiumoxid enthalten.

4. Katalysator nach Anspruch 1, worin in dem Träger das Polypropylen einen Aschegehalt von kleiner als 0,05 Gew.-% aufweist.

5. Katalysator nach Anspruch 1, worin in dem Träger ein keramisches Bindemittel zu dem extrudierbaren Gemisch in einer Menge zugesetzt wird, die von 0,01 bis 5 % des Gewichtes der keramischen Komponenten im Gemisch beträgt.

6. Katalysator nach Anspruch 1, worin der Träger 0,01 bis 5 Gew.-%, bezogen auf das Gesamtgewicht des gebrannten Trägers, einer Verbindung enthält, die aus der aus Erdalkalimetalloxid, Siliciumdioxid, Zirkoniumdioxid und deren Gemischen bestehenden Gruppe ausgewählt ist.

7. Katalysator nach Anspruch 1, worin der Träger zusätzlich 0,05 bis 5 Gew.-%, bezogen auf das Gewicht des gebrannten Trägers, Titandioxid enthält.

8. Katalysator nach Anspruch 1, worin das Silber im Bereich von 1 Gew.-% bis 40 Gew.-% des Gesamtkatalysators ausmacht und das Alkalimetall im Bereich von 10 Teilen pro Million bis 3000 Teilen pro Million, ausgedrückt als Metall und bezogen auf das Gewicht des Gesamtkatalysators, liegt.

9. Katalysator nach Anspruch 8, worin der Alkalimetallpromotor aus der aus Kalium, Rubidium, Cäsium, Lithium und deren Gemischen bestehenden Gruppe ausgewählt ist.

10. Katalysator nach Anspruch 9, worin der Promotor Cäsium ist.

11. Katalysator nach Anspruch 1, worin der Alkalimetallpromotor Cäsium und wenigstens ein weiteres Alkalimetall umfaßt.

12. Katalysator nach Anspruch 1, worin der Rheniumcopromotor aus der aus Schwefel, Molybdän, Wolfram, Chrom, Phosphor, Bor und deren Gemischen bestehenden Gruppe ausgewählt ist.

13. Verfahren zur Herstellung von Ethylenoxid, worin Ethylen in der Dampfphase mit einem sauerstoffhältigen Gas bei Ethylenoxidbildungsbedingungen bei einer Temperatur im Bereich von 180°C bis 330°C in Anwesenheit eines Katalysators nach einem der Ansprüche 1 bis 12 in Kontakt gebracht wird.

14. Verfahren zur Epoxidation von keinen Allylwasserstoff aufweisenden Olefinen, worin ein keinen Allylwasserstoff aufweisendes Olefin in der Dampfphase mit einem sauerstoffhältigen Gas bei Epoxidbildungsbedingungen bei einer Temperatur im Bereich von 75°C bis 325°C in Anwesenheit eines organischen Halogenids und eines Katalysators nach einem der Ansprüche 1 bis 12 in Kontakt gebracht wird.

## Revendications

1. Catalyseur qui convient à l'époxydation d'oléfines ne comportant pas d'hydrogène allylique, en particulier, de l'éthylène avec de l'oxygène moléculaire, lequel catalyseur comprend une quantité catalytiquement efficace d'argent, une quantité promotrice de métal alcalin, éventuellement, une quantité promotrice de rhénium et éventuellement, une quantité promotrice d'un promoteur conjoint ou copromoteur du rhénium, déposées sur un support préparé par un procédé qui comprend le mélange de composants céramiques particulaires avec de 0,5 à 50 parties en poids, sur base de 100 parties en poids des composants céramiques, d'un polypropylène, sous la forme d'une poudre, possédant un calibre moyen des particules inférieur à 400 micromètres et une teneur en cendre inférieure à 0,1% en poids et ensuite la calcination à une température qui suffit à éliminer par combustion le polymère organique synthétique le frittage du composant particulaire et la formation d'un support.

2. Catalyseur suivant la revendication 1, caractérisé en ce que, dans le support, le polypropylène est présent en une quantité qui varie de 1 à 40% en poids des composants céramiques.

3. Catalyseur suivant la revendication 1, caractérisé en ce que, dans le support, les composants céramiques comprennent au moins 90% en poids d'alpha-alumine.

4. Catalyseur suivant la revendication 1, caractérisé en ce que, dans le support, le polypropylène possède une teneur en cendre inférieure à 0,05% en poids.

5. Catalyseur suivant la revendication 1, caractérisé en ce que, dans le support, on ajoute une matière de liaison céramique au mélange extrudable en une proportion qui varie de 0,01 à 5% en poids des composants céramiques dans le mélange.

6. Catalyseur suivant la revendication 1, caractérisé en ce que le support comprend de 0,01 à 5% en poids, sur base du poids total du support calciné, d'un composé choisi dans le groupe formé par un oxyde de métal alcalino-terreux, le dioxyde de silicium, le dioxyde de zirconium et leurs mélanges.

7. Catalyseur suivant la revendication 1, caractérisé en ce que le support comprend en outre de 0,05% en poids à 5% en poids, sur base du poids du support calciné, d'oxyde de titane.

8. Catalyseur suivant la revendication 1, caractérisé en ce que la proportion d'argent varie de 1% en poids à 40% en poids du catalyseur total et la proportion de métal alcalin varie de 10 parties par million à 3000 parties par million, exprimées sous forme du métal, en poids du catalyseur total.

9. Catalyseur suivant la revendication 8, caractérisé en ce que l'on choisit le promoteur de métal alcalin dans le groupe constitué par le potassium, le rubidium, le césium, le lithium et leurs mélanges.

10. Catalyseur suivant la revendication 9, caractérisé en ce que le promoteur est le césium.

11. Catalyseur suivant la revendication 1, caractérisé en ce que le promoteur de métal alcalin précité comprend du césium plus au moins un métal alcalin supplémentaire.

12. Catalyseur suivant la revendication 1, caractérisé en ce que le copromoteur du rhénium est choisi dans le groupe formé par le soufre, le molybdène, le tungstène, le chrome, le phosphore, le bore et leurs mélanges.

13. Procédé de production de l'oxyde d'éthylène conformément auquel on met l'éthyléne en contact en phase vapeur avec un gaz contenant de l'oxygène dans des conditions formatrices d'oxyde d'éthylène, à une température qui varie de 180°C à 330°C et en présence d'un catalyseur suivant l'une quelconque des revendications 1 à 12.

14. Procédé d'époxydation d'oléfines ne comportant pas d'hydrogènes allyliques, caractérisé en ce que l'on met une oléfine ne comportant pas d'hydrogène allylique en contact, en phase vapeur, avec un gaz contenant de l'oxygène dans des conditions formatrices d'époxyde, à une température qui varie de 75°C à 325°C et en présence d'un halogénure organique et d'un catalyseur suivant l'une quelconque des revendications 1 à 12.
